# EUROPEAN PATENT SPECIFICATION

(11) **EP 3 712 249 B1**
(45) Date of publication and mention of the grant of the patent: **03.01.2024**
(21) Application number: 18883804.9
(22) Date of filing: 29.11.2018
(51) Int. Cl.: C12N 1/20, C12R 1/01, A23L 5/20, A62D 3/02, A62D 101/20

(54) **MYROIDES ODORATIMIMUS BIOCONTROL STRAIN FOR EFFICIENTLY DEGRADING AFLATOXIN, AND APPLICATION THEREOF**
BIOKONTROLLSTAMM MYROIDES ODORATIMIMUS ZUM EFFIZIENTEN ABBAU VON AFLATOXIN UND ANWENDUNG DAVON
SOUCHE DE MYROIDES ODORATIMIMUS POUR LA PROTECTION BIOLOGIQUE DE CULTURES POUR LA DÉGRADATION EFFICACE DE L'AFLATOXINE ET SON APPLICATION

(30) Priority: 29.11.2017 CN 201711225506; 23.11.2018 CN 201811409668
(43) Date of publication of application: 23.09.2020
(73) Proprietor: Oil Crops Research Institute, Chinese Academy of Agricultural Sciences, Wuhan, Hubei 430062 (CN)
(72) Inventor: ZHANG, Qi, Wuhan Hubei 430062 (CN); LI, Peiwu, Wuhan Hubei 430062 (CN); WANG, Tong, Wuhan Hubei 430062 (CN); DING, Xiaoxia, Wuhan Hubei 430062 (CN)
(74) Representative: Laufhütte, Dieter
(86) International application number: PCT/CN2018/118204
(87) International publication number: WO 2019/105416

(56) References cited:
- CN-A- 102 031 234
- CN-A- 103 981 132
- CN-A- 103 981 135
- CN-A- 105 925 513
- CN-A- 106 065 396
- CN-A- 106 381 277
- CN-A- 107 201 322
- US-A1- 2011 135 796
- LI T. ET AL: "Biodegradation of 3,4-Dichloroaniline by a Novel Strain LWD09 with Moderate Salinity Tolerance", WATER, AIR, AND SOIL POLLUTION, vol. 223, no. 6, 6 March 2012 (2012-03-06) , pages 3271-3279, XP035066624, ISSN: 1573-2932, DOI: 10.1007/S11270-012-1108-6
- MWAKINYALI S. E. ET AL: "Investigation and Characterization of Myroides odoratimimus Strain 3J2MO Aflatoxin B 1 Degradation", JOURNAL OF AGRICULTURAL AND FOOD CHEMISTRY, vol. 67, no. 16, 25 March 2019 (2019-03-25), pages 4595-4602, XP055753857, ISSN: 0021-8561, DOI: 10.1021/acs.jafc.8b06810
- LIU A. ET AL: "Decontamination of Aflatoxins by Lactic Acid Bacteria", CURRENT MICROBIOLOGY, vol. 77, no. 12, 26 September 2020 (2020-09-26), pages 3821-3830, XP037299761, ISSN: 0343-8651, DOI: 10.1007/S00284-020-02220-Y
- K. Raksha RAO: "Biological detoxification of Aflatoxin B1 by Bacillus licheniformis CFR1", Food Control, vol. 71, 28 June 2016 (2016-06-28), pages 234-241, XP029666120,

## Description

### BACKGROUND

### Technical Field

The disclosure belongs to the field of microbes and specifically relates to a Myroides odoratimimus biocontrol strain for efficiently degrading aflatoxin and an application thereof.

### Description of Related Art

Aspergillus flavus is a pathogenic fungus that can produce a type of strongly carcinogenic and highly toxic fungal toxin, aflatoxin, including B, G, and M families, wherein B 1 is the most common and the most toxic. Aflatoxin may widely pollute food crops such as peanuts, corn, etc. and seriously threaten the health of human and livestock, causing great economic losses. Therefore, it is urgent to strengthen the treatment of Aspergillus flavus and toxin pollution.

The traditional methods of removing aflatoxin B 1 (AFB1) mainly include adsorption, extraction, heat treatment, radiation treatment, acid-base treatment, and redox treatment, which are sometimes time and effort consuming, have low detoxification rate, and may easily cause the loss of nutrients. However, the biological method has the advantages of safety, high efficiency, and lasting. Therefore, it is of great significance for the agricultural industry and economic benefits of China to strengthen the research on aflatoxin biological treatment.

Bacterial and metabolite biodegradation of aflatoxin is not widely reported. Stenotrophomonas maltophilia screened by cumoric medium as applied by Guan Shu, Li Junxia, et al. can degrade aflatoxin with a degradation rate of 82.5%. Wang Ning et al. studied an orange-red myxococcus strain from gray langur feces and discovered that the degradation rate of AFB1 is 78.2% under the optimal culture conditions. In the prior art, there have been no reports of the use of Myroides odoratimimus to degrade aflatoxin.

CN 105 925 513 A discloses Flavobacterium strain CICC 20907 as microbial agent for degrading aflatoxin B1. Li T., et al., "Biodegredation of 3,4-Dichloroaniline by a Novel Strain LWD09 with Moderate Salinity Tolerance", Water, Air, and Soil Pollution, Vol. 223, No. 6, 6 March 2021, pages 3271-3279 discloses Myriodes odoratimimus strain LWD09 for biodegradation of 3,4-dichloroanaline.

### SUMMARY

The technical problem to be solved by the invention is to provide a Myroides odoratimimus biocontrol strain 3J2MO for efficiently degrading aflatoxin and an application thereof in view of the deficiencies of the prior art. The Myroides odoratimimus biocontrol strain 3J2MO in the invention can significantly degrade aflatoxin.

In order to solve the above technical problem, the technical solution adopted by the invention is as follows:

The Myroides odoratimimus biocontrol strain 3J2MO was preserved at the China Center for Type Culture Collection (referred to as CCTCC) on June 13, 2017 with preservation address: Wuhan University, Wuhan, China and preservation number CCTCC No. M 2017329.

In the invention, 100 µL of the soil after gradient dilution collected from the peanut field in Huangpi, Hubei is smeared in a lysogeny broth (LB) solid culture medium for culturing. Grown bacteria are selected with an inoculating loop and transferred to a fresh LB solid culture medium for streak plate. A single colony is selected after multiple transfers. A strain 3J2MO with significant degradation effect on aflatoxin is screened through the aflatoxin degradation experiment and is preserved at the China Center for Type Culture Collection (referred to as CCTCC) with preservation address: Wuhan University, Wuhan, China and preservation number CCTCC No. M 2017329. The type of the strain is identified using 16S rDNA specificity amplification technology combined with morphological characteristics, and physiological and biochemical experiments. The result shows that the strain is a Myroides odoratimimus strain, belonging to actinomycetes, brevibacterium.

**Table 1: Main biological characteristics of Myroides odoratimimus 3J2MO:**

| Gram stain | Oxygen | Spore | Catalase | Culture temperature | Culture time | MR test | Nitrate reduction | Catalase | Acid-fast stain |
|---|---|---|---|---|---|---|---|---|---|
| Positive | Facultative anaerobic | Not formed | Positive | 20-30°C | 12-24 hours | Positive | Positive | Positive | Negative |

A preparation for degrading aflatoxin is provided, which includes a ferment of the Myroides odoratimimus 3J2MO.

According to the solution, the preparation form of the preparation is liquid, spraying powder, dry wettable powder, or dry wettable granule.

According to the solution, the preparation is liquid and the final concentration of Myroides odoratimimus 3J2MO in the preparation is (1-9) × 10⁷ CFU/mL.

According to the solution, the preparation is a fermentation broth of Myroides odoratimimus 3J2MO and the final concentration of Myroides odoratimimus 3J2MO in the fermentation broth of Myroides odoratimimus 3J2MO is (1-9) × 10⁷ CFU/mL.

According to the solution, the preparation method of the fermentation broth of Myroides odoratimimus 3J2MO is as follows. Myroides odoratimimus 3J2MO is activated in an LB plate and cultured in an incubator at 28°C for 24 hours. A single colony of Myroides odoratimimus is selected with a needle, transferred to a LB liquid culture medium, and shaking cultured for 12 hours to 24 hours. 1% to 3% of a culture broth is absorbed, transferred to a fresh LB liquid culture medium, and shaking cultured for 12 hours to 4 hours to obtain a fermentation broth of an antagonize strain of Myroides odoratimimus 3J2MO.

A method for degrading aflatoxin is provided and the specific application method is as follows. A preparation for degrading aflatoxin is coated or sprayed onto the surface of a biological sample or mixed with the biological sample for degradation of aflatoxin to prevent and cure aflatoxin pollution of the biological sample.

A method for degrading aflatoxin is provided and the specific application method is as follows. A fermentation broth of Myroides odoratimimus 3J2MO is coated or sprayed onto the surface of a biological sample or mixed with the biological sample for degradation of aflatoxin to prevent and cure aflatoxin pollution of the biological sample.

The beneficial effects of the invention:
The invention provides the Myroides odoratimimus strain 3J2MO with good degradation effect on aflatoxin from the soil for the first time.

### DETAILED DESCRIPTION OF DISCLOSED EMBODIMENTS

In the invention, 100 µL of the soil after gradient dilution collected from the peanut field in Huangpi, Hubei is smeared in a lysogeny broth (LB) solid culture medium for culturing. Grown bacteria are selected with an inoculating loop and transferred to a fresh LB solid culture medium for streak plate. A single colony is selected after multiple transfers. A strain 3J2MO with significant degradation effect on aflatoxin is screened through the aflatoxin degradation experiment and is preserved at the China Center for Type Culture Collection (referred to as CCTCC) with preservation number CCTCC No. M 2017329.

### Embodiment 1:

1) Myroides odoratimimus 3J2MO is activated in an LB plate and cultured in an incubator at 28°C for 24 hours. A single colony of activated Myroides odoratimimus is selected with a needle, transferred to a triangular flask containing 15 mL of LB liquid culture medium, and shaking cultured at 28°C and 200r·min⁻¹ for 12 hours. 1% of a culture broth is absorbed, transferred to a triangular flask containing 15mL of LB liquid culture medium, and shaking cultured at 28°C and 200r·min⁻¹ for 12 hours to obtain a fermentation broth of an antagonize strain.
2) An aflatoxin B 1 standard solution is added to a fermentation broth of Myroides odoratimimus (with final concentration of 1×10⁷ CFU/mL) to be cultured in the LB culture medium together at 28°C and 200 rpm for 5 days with 3 repetitions per treatment.
3) The content of aflatoxin B 1 in the culture broth is measured (Table 2).

**Table 2 Degradation effect of biocontrol bacteria on aflatoxin**

| Treatment | AFB1 | AFB 1+CCTCC No. M 20177329 |
|---|---|---|
| AFB 1 toxin content (ng/ml) | 39.69±3.90 | 1.61±0.16 |

It can be seen from the experimental result that the degradation rate of the Myroides odoratimimus strain CCTCC No. M 2017329 on aflatoxin is about 95.61%, indicating that the strain has the ability to degrade aflatoxin.

### Embodiment 2:

Peanut grains obtained from Hubei peanut field are ground into powder and 0.5 g of peanut powder is weighed into a petri dish. 500 µL of spore solution of toxin-producing Aspergillus flavus (5×10⁵/mL) is added and cultured in an incubator at 28°C for 7 days.

A fermentation broth of 500 µL of CCTCC No. M 20177329 strain is added to the peanut powder filled with spores, the same amount of LB culture medium is used as a control, and cultured in the culture medium at 28°C for 5 days with 3 repetitions in the experiment.
15 mL of 70% methanol water is added and placed in a shaker for 30 minutes after vortex. 8 mL of ultrapure water is added to 3 mL of supernatant to vortex and centrifuge;
8 mL of supernatant is taken and the content of aflatoxin B 1 is determined using immunoaffinity column, high-performance liquid chromatography (HPLC) method (Table 3), with 3 repetitions in the experiment.

**Table 3 Treatment effect of biocontrol bacteria on Aspergillus flavus polluted peanut**

| Treatment | AFB1 | AFB1+CCTCC No. M 20177329 |
|---|---|---|
| AFB 1 toxin content (ng/ml) | 111.68±9.05 | 1.39±0.36 |

It can be seen from the experimental result that the prevention and control rate of CCTCC No. M 2017329 grain on toxin production by Aspergillus flavus on peanut is about 98.76%, indicating that the strain has a good treatment effect on Aspergillus flavus polluted peanut.

### Embodiment 3:

1) Myroides odoratimimus 3J2MO is activated in an LB plate and cultured in an incubator at 28°C for 24 hours. A single colony of activated Myroides odoratimimus is selected with a needle, transferred to a triangular flask containing 15 mL of LB liquid culture medium, and shaking cultured at 28°C and 200 r·min⁻¹ for 12 hours. 1% of a culture broth is absorbed, transferred to a triangular flask containing 15mL of LB liquid culture medium, and shaking cultured at 28°C and 200 r·min⁻¹ for 12 hours to obtain a fermentation broth of an antagonize strain.
2) An aflatoxin B2 standard solution is added to a fermentation broth of Myroides odoratimimus (with final concentration of 1×10⁷ CFU/mL) to be cultured in the LB culture medium together at 28°C and 200 rpm for 5 days with 3 repetitions per treatment.
3) The final content of aflatoxin B2 in the culture medium is measured and the degradation rate is calculated.

After calculation of the experimental result, the degradation rate of the Myroides odoratimimus CCTCC No. M2017329 strain on aflatoxin B2 is 96.23%, indicating that the strain has the ability to degrade aflatoxin B2.

### Embodiment 4:

1) Myroides odoratimimus 3J2MO is activated in an LB plate and cultured in an incubator at 28°C for 24 hours. A single colony of activated Myroides odoratimimus is selected with a needle, transferred to a triangular flask containing 15mL of LB liquid culture medium, and shaking cultured at 28°C and 200 r·min⁻¹ for 12 hours. 1% of a culture broth is absorbed, transferred to a triangular flask containing 15mL of LB liquid culture medium, and shaking cultured at 28°C and 200 r·min⁻¹ for 12 hours to obtain a fermentation broth of an antagonize strain.
2) An aflatoxin G1 standard solution is added to the fermentation broth of Myroides odoratimimus (with final concentration of 1×10⁷ CFU/mL) to be cultured in the LB culture medium together at 28°C and 200rpm for 5 days with 3 repetitions per treatment.
3) The final content of aflatoxin G1 in the culture medium is measured and the degradation rate is calculated.

After calculation of the experimental result, the degradation rate of the Myroides odoratimimus CCTCC No. M2017329 strain on aflatoxin G1 is 96.73%, indicating that the strain has the ability to degrade aflatoxin G1.

### Embodiment 5:

1) Myroides odoratimimus 3J2MO is activated in an LB plate and cultured in an incubator at 28°C for 24 hours. A single colony of activated Myroides odoratimimus is selected with a needle, transferred to a triangular flask containing 15mL of LB liquid culture medium, and shaking cultured at 28°C and 200 r·min⁻¹ for 12 hours. 1% of a culture broth is absorbed, transferred to a triangular flask containing 15mL of LB liquid culture medium, and shaking cultured at 28°C and 200 r·min⁻¹ for 12 hours to obtain a fermentation broth of an antagonize strain.
2) An aflatoxin G2 standard solution is added to the fermentation broth of Myroides odoratimimus (with final concentration of 1×10⁷ CFU/mL) to be cultured in the LB culture medium together at 28°C and 200 rpm for 5 days with 3 repetitions per treatment.
3) The final content of aflatoxin G2 in the culture medium is measured and the degradation rate is calculated.

After calculation of the experimental result, the degradation rate of the Myroides odoratimimus CCTCC No. M2017329 strain on aflatoxin G2 is 97.55%, indicating that the strain has the ability to degrade aflatoxin G2.

### Embodiment 6:

1) Myroides odoratimimus 3J2MO is activated in an LB plate and cultured in an incubator at 28°C for 24 hours. A single colony of activated Myroides odoratimimus is selected with a needle, transferred to a triangular flask containing 15mL of LB liquid culture medium, and shaking cultured at 28°C and 200 r·min⁻¹ for 12 hours. 1% of a culture broth is absorbed, transferred to a triangular flask containing 15mL of LB liquid culture medium, and shaking cultured at 28°C and 200 r·min⁻¹ for 12 hours to obtain a fermentation broth of an antagonize strain.
2) An aflatoxin M1 standard solution is added to the fermentation broth of Myroides odoratimimus (with final concentration of 1×10⁷ CFU/mL) to be cultured in the LB culture medium together at 28°C and 200 rpm for 5 days with 3 repetitions per treatment.
3) The final content of aflatoxin M1 in the culture medium is measured and the degradation rate is calculated.

After calculation of the experimental result, the degradation rate of the Myroides odoratimimus CCTCC No. M2017329 strain on aflatoxin M1 is 97.55%, indicating that the strain has the ability to degrade aflatoxin M1.

### Embodiment 7:

The same method as Patent Document CN 105925513 A is adopted. The degradation effects of flavobacterium with preservation number CICC 20907 in the patent document and the Myroides odoratimimus CCTCC No. M2017329 strain of the present invention on aflatoxin B1 are compared. The comparison results are shown in Table 4. The results show that the degradability of Myroides odoratimimus CCTCC No. M2017329 on aflatoxin B1 provided by the present invention far exceeds the degradability of flavobacterium CICC 20907 on aflatoxin B1 in Patent Document CN 105925513 A.

**Table 4 Comparison of degradability of present invention and flavobacterium CICC 20907 strain in Patent 105925513 A on aflatoxin B1**

| Degradation rate of Myroides odoratimimus CCTCC No. M2017329 on aflatoxin B 1 | Degradation rate of flavobacterium CICC 20907 on aflatoxin B1 in Patent CN 105925513 A |
|---|---|
| Test 1: 97.2% | Embodiment 1: 45.7% |
| Test 2: 98.3% | Embodiment 2: 46.7% |
| Test 3: 97.1% | Embodiment 3: 45.8% |
| Test 4: 96.7% | Embodiment 4: 47.1% |

Combining Embodiments 1, 2, 3, 4, 5, 6, and 7, the Myroides odoratimimus CCTCC No. M2017329 strain of the invention has very strong degradability on all five types of common aflatoxins. After researching domestic and foreign citations, a strain with such strong degradability on five types of common aflatoxins has not been reported domestically and abroad.

## Claims

1. A Myroides odoratimimus biocontrol strain 3J2MO, wherein the Myroides odoratimimus biocontrol strain 3J2MO is preserved at the China Center for Type Culture Collection (referred to as CCTCC) on June 13, 2017 with preservation number CCTCC No. M 2017329.

2. A preparation for degrading aflatoxin, wherein the preparation is a ferment comprising the Myroides odoratimimus biocontrol strain 3J2MO according to claim 1.

3. The preparation according to claim 2, wherein a preparation form of the preparation is liquid, spraying powder, dry wettable powder, or dry wettable granule.

4. The preparation according to claim 2, wherein the preparation is liquid and the final concentration of the Myroides odoratimimus biocontrol strain 3J2MO in the preparation is in the range of 1-9 × 10⁷ CFU/mL.

5. The preparation according to claim 2, wherein the preparation is a fermentation broth comprising the Myroides odoratimimus 3J2MO and a final concentration of Myroides odoratimimus 3J2MO in the fermentation broth is in the range of 1-9 × 10⁷ CFU/mL.

6. A method for making the preparation according to claim 5, comprising: activating Myroides odoratimimus biocontrol strain 3J2MO in a lysogeny broth 20 (LB) plate to be cultured in an incubator at 28°C for 24 hours; selecting a single colony of Myroides odoratimimus with a needle to be transferred to a liquid culture medium and shaking cultured for 12 hours to 24 hours; absorbing 1% to 3% of a culture broth to be transferred to a fresh LB liquid culture medium and shaking cultured for 12 hours to 24 hours; obtaining the fermentation broth of an antagonize strain of Myroides odoratimimus 3J2MO.

7. A method for degrading aflatoxin, wherein the preparation according to claim 2 or claim 5 is coated or sprayed onto a surface of a biological sample or mixed with the biological sample for degradation of aflatoxin.

## Patentansprüche

1. Myroides odoratimimus-Biokontrollstamm 3J2MO, wobei der Myroides odoratimimus-Biokontrollstamm 3J2MO am 13. Juni 2017 im China Center for Type Culture Collection (bezeichnet als CCTCC) mit der Konservierungsnummer CCTCC Nr. M 2017329 konserviert wird.

2. Präparat zum Abbau von Aflatoxin, wobei das Präparat ein Ferment ist, das den Myroides odoratimimus-Biokontrollstamm 3J2MO nach Anspruch 1 umfasst.

3. Präparat nach Anspruch 2, wobei eine Präparatform des Präparats Flüssigkeit, Sprühpulver, trockenes benetzbares Pulver oder trockenes benetzbares Granulat ist.

4. Präparat nach Anspruch 2, wobei das Präparat Flüssigkeit ist und die Endkonzentration des Myroides odoratimimus-Biokontrollstammes 3J2MO in dem Präparat im Bereich von 1-9 × 10⁷ CFU/mL liegt.

5. Präparat nach Anspruch 2, wobei das Präparat eine Fermentationsbrühe ist, die den Myroides odoratimimus-Biokontrollstamm 3J2MO umfasst, und wobei eine Endkonzentration von Myroides odoratimimus-Biokontrollstamm 3J2MO in der Fermentationsbrühe im Bereich von 1-9 × 10⁷ CFU/mL liegt.

6. Verfahren zur Herstellung des Präparats nach Anspruch 5, wobei es umfasst: Aktivieren des Myroides odoratimimus-Biokontrollstammes 3J2MO in einer Lysogeniebrühe 20 (LB)-Platte, um in einem Inkubator bei 28°C für 24 Stunden kultiviert zu werden; Auswählen einer einzelnen Kolonie von Myroides odoratimimus-Biokontrollstamm 3J2MO mit einer Nadel, die in ein flüssiges Kulturmedium übertragt und für 12 bis 24 Stunden geschüttelt kultiviert wird; Absorbieren von 1 bis 3 % einer Kulturbrühe, die in ein frisches flüssiges LB-Kulturmedium übertragt und für 12 bis 24 Stunden geschüttelt kultiviert wird; und Erhalten der Fermentationsbrühe eines Myroides odoratimimus-Antagonistenstammes 3J2MO.

7. Verfahren zum Abbau von Aflatoxin, wobei das Präparat nach Anspruch 2 oder 5 auf eine Oberfläche einer biologischen Probe aufgetragen oder aufgesprüht oder mit der biologischen Probe gemischt wird, um Aflatoxin abzubauen.

## Revendications

1. Souche de biocontrôle 3J2MO de Myroides odoratimimus, dans laquelle la souche de biocontrôle 3J2MO de Myroides odoratimimus est conservée au China Center for Type Culture Collection (CCTCC) le 13 juin 2017 sous le numéro de conservation CCTCC M2017329.

2. Préparation de dégradation de l'aflatoxine, dans laquelle la préparation est un ferment comprenant la souche de biocontrôle 3J2MO de Myroides odoratimimus selon la revendication 1.

3. Préparation selon la revendication 2, dans laquelle la préparation est sous forme de liquide, de poudre pulvérisée, de poudre mouillable sèche ou granule mouillable sec.

4. Préparation selon la revendication 2, dans laquelle la préparation est sous forme de liquide et la concentration finale de la souche de biocontrôle 3J2MO de Myroides odoratimimus dans la préparation est dans une plage de 1 à 9 × 10⁷ CFU/mL.

5. Préparation selon la revendication 2, dans laquelle la préparation est un bouillon de fermentation comprenant la souche de biocontrôle 3J2MO de Myroides odoratimimus et une concentration finale de la souche de biocontrôle 3J2MO de Myroides odoratimimus dans le bouillon de fermentation est dans une plage de 1 à 9 × 10⁷ CFU/mL.

6. Procédé de production de la préparation selon la revendication 5, comprenant : activer la souche de biocontrôle 3J2MO de Myroides odoratimimus dans une plaque de bouillon de lysogénie 20 (LB) et cultiver dans un incubateur à 28 °C pendant 24 heures ; sélectionner une seule colonie de la souche de biocontrôle 3J2MO de Myroides odoratimimus à l'aide d'une aiguille et la transférer dans un milieu de culture liquide pour cultiver sous agitation pendant 12 à 24 heures ; extraire 1 % à 3 % d'un bouillon de culture et le transférer dans un milieu de culture liquide LB frais sous agitation pendant 12 à 24 heures ; et obtenir le bouillon de fermentation d'une souche antagonique de Myroides odoratimimus 3J2MO.

7. Procédé de dégradation de l'aflatoxine, dans lequel la préparation selon la revendication 2 ou la revendication 5 est appliquée ou pulvérisée sur une surface d'un échantillon biologique ou mélangée à l'échantillon biologique pour la dégradation de l'aflatoxine.
